# EUROPEAN PATENT APPLICATION

(11) **EP 2 612 676 A1**
(43) Date of publication of application: **10.07.2013**
(21) Application number: 11821119.2
(22) Date of filing: 31.08.2011
(51) Int. Cl.: A61K 38/19, A61P 7/00

(54) **USE OF G-CSF DIMER IN THE TREATMENT OF NEUTROPENIA**

(30) Priority: 31.08.2010 CN 201010268290
(71) Applicant: Generon (Shanghai) Corporation Ltd., Shanghai 201203 (CN)
(72) Inventor: YAN, Xiaoqiang, Shanghai 201203 (CN); HUANG, Zhihua, Shanghai 201203 (CN); YANG, Hongzhou, Shanghai 201203 (CN); SUN, Bill, N.C., Shanghai 201203 (CN); HUANG, Yuliang, Shanghai 201203 (CN)
(74) Representative: Spencer, Matthew Peter
(86) International application number: PCT/CN2011/079143
(87) International publication number: WO 2012/028093

(57) **Abstract**

This invention relates to a use of G-CSF dimer in the treatment of neutropenia. In particular, the recombinant human G-CSF of the present invention can enhance the differentiation and development of neutrophils in animal, and thus effectively reduce the severity of the severe neutropenia and shorten the time of severe neutropenia for the post-chemotherapy cancer patients. Serum half-life of G-CSF dimer of this invention is prolonged and the biological activity thereof is increased, providing a better effect in the treatment of neutropenia.

## Description

### REFERENCE TO SEQUENCE LISTING

The hard copy of the sequence listing submitted herewith and the corresponding computer readable form are both incorporated herein by reference in their entireties.

### FIELD OF INVENTION

This invention relates to the area of biological and medical technologies, in particular, this invention relates to the use of recombinant human G-CSF (rhG-CSF) dimer in the treatment of neutropenia.

### BACKGROUND OF INVENTION

Human granulocyte colony-stimulating factor (G-CSF) is a glycoprotein having 204 amino acids with 30 amino-acid signal peptides. Mature G-CSF protein, having 18-20 kDa in molecular weight, is composed of 174 amino acids without signal peptides and secreted out of the cells. Human cells mainly responsible for such secretion are monocytes, fibroblasts, and endothelial cells.

There are three main biological functions for G-CSF in an living organism, namely: 1. acting on neutrophil precursor cells and myeloid stem cells to drive the differentiation, proliferation, and maturation of neutrophils; 2. activating mature neutrophils to participate in immune response; and 3. synergizing with other hematopoietic growth factors such as stem cell factor, Flt-3 ligand, and GM-CSF to perform hematopoietic functions.

G-CSF receptor (G-CSFR) is proven to exist in bone marrow hematopoietic stem cell Sca⁺Lin⁻Th1^{low} precursor cell CD34⁺, committed granulocyte precursor cell, and mature neutrophil. Human G-CSFR is a single-chain specific receptor having a high affinity to G-CSF and is composed of 812 amino acids.

Tamada et al. obtained the crystalline structure of the G-CSF:G-CSFR complex and the stoichiometry of G-CSF:G-CSFR complex was shown as a 2:2 ratio by the 2.8 angstrom diffraction analysis (PNAS, 2008, Vol. 103: 3135-3140). In other words, each complex has two G-CSF molecules and two G-CSFR molecules. Each G-CSF molecule binds to one receptor to form a G-CSF-receptor complex and when two G-CSF-receptor complexes are brought to close proximity, a 2:2 dimer is formed as a result of this interaction. Under this circumstance, the carboxyl terminal of the G-CSF receptor is then able to activate the downstream signal molecules JAK2 (Janus tyrosine kinases). Consequently, JAK2 actives STAT3 to switch on the transcriptional genes to stimulate the cell proliferation.

Neutropenia is characterized by a neutrophil count in the peripheral blood of lower than 1.8x10⁹/L for an adult and 1.5x10⁹/L for a child. Neutropenia is often a precursor of infection: the lower the neutrophil count is, the higher the risk of infection is.

The guideline used to classify neutropenia is shown as below:

| Neutropenia | Neutrophil Count | Risk of Infection |
|---|---|---|
| Mild | 1.0~1.8×10⁹/L | Minimal |
| Moderate | 0.5~1.0×10⁹/L | Increasing |
| Severe | <0.5×10⁹/L | Severe |

The frequency and severity of infection caused by neutropenia are also influenced by other factors, such as: the integrity of the mucosa and skin, immunoglobulin, lymphocytes, monocytes, the function and level of the complement system, etc.

According to the cause of neutropenia, the common clinical neutropenia can be divided into the following categories: disorder of hematopoietic system generation that are caused by secondary factors such as drugs, radiation, chemical reagents and infection; changes of *in vivo* distribution and circulation, increased utilization and turnover. The severity of chemotherapy-induced neutropenia in tumor patients generally depends on the dosage of chemotherapy, and the repeated use of chemotherapy may have a cumulative effect on neutropenia. A main clinical consequence of neutropenia is infected complication. Most of the infections in those patients are mainly caused by aerobic bacteria, including Gram-negative bacteria (*Escherichia coli, Klebsiella pheumoniae* and *Pseudomonas aeruginosa),* Gram-positive bacteria (Staphylococci, *α*-hemolytic Streptococci, and *Straphylococcus aureus*) and fungi.

Cytotoxic-chemotherapy is still one of the major treatments of cancer. The biggest disadvantage of chemotherapy treatment is that this treatment would indiscriminately kill healthy cells with rapid proliferation and differentiation together with tumor cells. The toxicity caused by chemotherapy is mainly expressed in the hematopoietic system that is neutropenia which is clinically known as chemotherapy-induced neutropenia.

Neutropenia may delay the next treatment cycle, which directly impacts on the therapeutic effects of chemotherapy. A severe neutropenia, i.e. the absolute neutrophil count (ANC) is lower than 0.5 x 10⁹/L, can cause infection in patient, organ failure and even threaten the life of the patient. Recombinant human granulocyte colony-stimulating factor (rhG-CSF) has been widely used in chemotherapy-induced and/or radiotherapy-induced neutropenia as a standard supportive therapy for chemotherapy-treated cancer patients.

There are two main categories of rhG-CSF used for therapy available in the market. The first category comprises recombinant proteins expressed by *E. coli* comprising 175 amino acids with 19 kD in molecular weight and the amino terminus thereof is methionine (Filgrastim); recombinant proteins produced by the mammalian cell CHO comprising 174 amino acids and modified by glycosylation. This category of rhG-CSF is short-acting and requires multiple injections daily or weekly. The second category comprises Filgrastim with pegylation (20 kD-PEG) modification on the N terminal of the protein molecule thereof. The molecular weight of the modified Pegfilgrastim is doubled, which reduces the renal excretion rate, increases the half-life of Filgrastim from 3.5 hours to 15-80 hours and facilitates the clinical use. The rhG-CSF used in both categories is G-CSF monomer.

However, the short-acting Filgrastim and the long-acting Pegfilgrastim currently used in clinical application still cannot meet the needs of patients. In 2008, Sierra et. al. compared the effects of Filgrastim and the Pegfilgrastim in the treatment of neutropenia in chemotherapy-treated acute leukemia patients *(*BMC Cancer 2008, 8:195). The study was designed as a randomized double-blind clinical trial. Patients with acute myeloid leukemia were treated with chemotherapy Induction I, receiving a chemotherapeutic agent of Idarubicin 12 mg/M² from Day 1 to Day 3, and a chemotherapeutic agent of Cytarabine 100 mg/M² from Day 1 to Day 7, twice per day. The patients were randomly divided into two groups at Days 6-8. One group was treated with Filgrastim 5 *µ*g/kg/day (n=41) while the other group was treated with Pegfilgrastim 6.0 mg/week (n=42). The results showed that, after completion of chemotherapy, all patients suffered from severe neutropenia that lasted around three weeks starting from 3-4 days upon the use of rhG-CSF therapy. Moreover, there was no significant difference in the efficacy of two groups receiving two different rhG-CSF treatments. This suggested that the mere extension of half-life seems to be sufficient to obtain a satisfactory therapeutic effect.

Therefore, there is an urgent need in the art to develop more effective drugs for treating neutropenia, in order to effectively reduce the severity of the neutropenia and / or shorten the time of severe neutropenia.

### SUMMARY OF INVENTION

In the light of the foregoing background, it is an object of the present invention to provide an alternate drug for the treatment of neutropenia with improved efficacy and the use thereof

Accordingly, the present invention, in one aspect, provides a use of human granulocyte colony-stimulating factor (G-CSF) dimer in the manufacture of a drug for treating neutropenia.

In an exemplary embodiment of the present invention, the neutropenia comprises a condition in which neutropenia induced by chemotherapy and/or radiotherapy.

In another exemplary embodiment, the neutropenia is severe neutropenia.

In another implementation, the human G-CSF (hG-CSG) dimer is shown as formula (I):

(I) M1-L-M2

wherein

M1 is a first human G-CSF monomer;

M2 is a second human G-CSF monomer ; and

L is a linker connecting the first monomer and the second monomer and disposed there between.

The G-CSF dimer retains the biological activity of a G-CSF monomer and has a serum half-life of at least twice of the half-life of either the first or the second monomer.

In an exemplary embodiment of the present invention, the linker L is selected from the group consisting of:

i). a short peptide (or a connecting peptide) comprising 3 to 50 (or 5 to 50) amino acids; and

ii). a polypeptide of formula (II):

(II) -Z-Y-Z-

wherein

Y is a carrier protein;

Z is null, or a short peptide(s) comprising 1 to 30 amino acids.

"-" is a chemical bond or a covalent bond.

In another exemplary embodiment, the first monomer and the second monomer are of the same entity.

In another exemplary embodiment, the first monomer and the second monomer are of the different entities.

In an exemplary embodiment, the biological activity includes:

(a). acting on neutrophil precursor cells and myeloid stem cells to drive the differentiation, proliferation, and maturation of neutrophils; and

(b). activating mature neutrophils to participate in immune response.

In another exemplary embodiment, the carrier protein is albumin or Fc fragment of human IgG.

In another exemplary embodiment, at least the first to the fourth amino acids of the hinge region are missing in the Fc fragment of human IgG and at least two cysteine residues are retained in the Fc fragment.

In another exemplary embodiment, the carrier protein is formed by the connection of two Fc fragments of IgG via disulfide bonds. In another exemplary embodiment, there are 2-3 disulfide bonds between the two Fc fragments.

In another exemplary embodiment, the "-" is a peptide bond.

In one exemplary embodiment, the serum half-life of the G-CSF dimer is at least three, five, or ten times of the half-life of the first and/or the second monomer.

In another exemplary embodiment, the G-CSF dimer is formed by two monomers in which the monomer comprises an amino acid sequence selected from a group consisting of SEQ ID NO: 2-6.

In another aspect of the present invention, a G-CSF dimer of formula (I) is provided:

(I) M1-L-M2

wherein

M1 is a first G-CSF monomer ;

M2 is a second G-CSF monomer ; and

L is a linker connecting the first monomer and the second monomer and disposed there between.

Also, the G-CSF dimer retains the biological activity of G-CSF monomer and has a serum half-life of at least twice of the half-life of either the first or the second monomer.

In another exemplary embodiment, the carrier protein is albumin or Fc fragment of human IgG.

In another exemplary embodiment, a method of preparing the G-CSF dimer comprises the following steps of:

a). transforming mammalian cells with an expression vector comprising a DNA sequence encoding G-CSF-Fc complex;

b). culturing the transformed mammalian cells for expressing an expression product comprising the G-CSF-Fc complex, the G-CSF dimer and the polymer thereof; and

c). isolating and purifying the G-CSF dimer.

In the third aspect of the present invention, a pharmaceutical composition is provided, comprising a human G-CSF dimer as described in the second aspect of the present invention and a pharmaceutically acceptable carrier.

In another exemplary embodiment, said pharmaceutical composition basically does not comprise any human G-CSF monomer. In a preferred exemplary embodiment, a weight ratio of human G-CSF dimer to human G-CSF monomer is ≥ 20:1; in an even preferred embodiment, the weight ratio thereof is ≥ 30:1; and in the most preferred embodiment, the weight ratio thereof is ≥ 50:1.

It is clear for a skilled person in the art that, the technical features mentioned above and discussed in the examples below of the present invention could combine with each other to result in a new or even better technical solution. Hence this invention should not be construed as limited to the embodiments set forth herein.

### BRIEF DESCRIPTION OF FIGURES

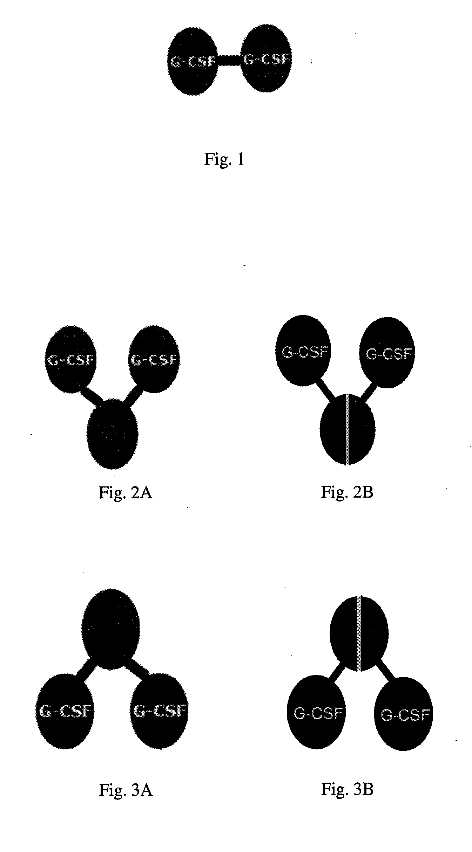

Fig. 1 illustrates the structure of a G-CSF dimer according to one embodiment of the present invention. In the figure, "-" represents the linker and the oval-shaped object labeled with "G-CSF" represents a G-CSF monomer.

An amino acid sequence of the G-CSF dimer is shown in SEQ ID NO: 1, in which the amino acid residues 1-174 represent a G-CSF monomer, the amino acid residues 175-190 represent a linker, and the amino acid residues 191-364 represent another G-CSF monomer.

Fig. 2A and 2B illustrate the structure of a G-CSF dimer according to one embodiment of the present invention. In the figure, "-" represents the linker and the oval-shaped object labeled with "G-CSF" represents a G-CSF monomer. The oval-shaped object labeled with "C" represents a carrier protein in which the G-CSF monomer is disposed at the N-terminal of the carrier protein. The coupling of two Fc fragments via disulfide bond is also shown in Fig. 2B.

An amino acid sequence of a G-CSF monomer with Fc fragment to form a G-CSF dimer is shown in SEQ ID NO: 2, in which the amino acid residues 1-174 represent a G-CSF monomer, the amino acid residues 175-190 represent a linker, and the amino acid residues 191-418 represent an Fc fragment of human IgG2. A G-CSF dimer is formed by the coupling of the Fc fragments present in the two G-CSF monomers.

An amino acid sequence of a G-CSF monomer with Fc fragment to form a G-CSF dimer is shown in SEQ ID NO: 3, in which the amino acid residues 1-174 represent a G-CSF monomer, the amino acid residues 175-180 represent a linker, and the amino acid residues 191-408 represent an Fc fragment of human IgG2. A G-CSF dimer is formed by the coupling of the Fc fragments present in the two G-CSF monomers.

Fig. 3A and 3B illustrate the structure of a G-CSF dimer according to one embodiment of the present invention. In the figure, "-" represents the linker and the oval-shaped object labeled with "G-CSF" represents a G-CSF monomer. The oval-shaped object labeled with "C" represents a carrier protein in which the G-CSF monomer is disposed at the C-terminal of the carrier protein. The coupling of two Fc fragments via disulfide bond is also shown in Fig. 3B.

An amino acid sequence of a G-CSF monomer with Fc fragment to form a G-CSF dimer is shown in SEQ ID NO: 4, in which the amino acid residues 1-228 represent an Fc fragment of human IgG2, the amino acid residues 229-244 represent a linker, and the amino acid residues 245-418 represent a G-CSF monomer. A G-CSF dimer is formed by the coupling of the Fc fragments present in the two G-CSF monomers.

An amino acid sequence of a G-CSF monomer with Fc fragment to form a G-CSF dimer is shown in SEQ ID NO: 5, in which the amino acid residues 1-228 represent an Fc fragment of human IgG2, the amino acid residues 229-234 represent a linker, and the amino acid residues 235-418 represent a G-CSF monomer. A G-CSF dimer is formed by the coupling of the Fc fragments present in the two G-CSF monomers.

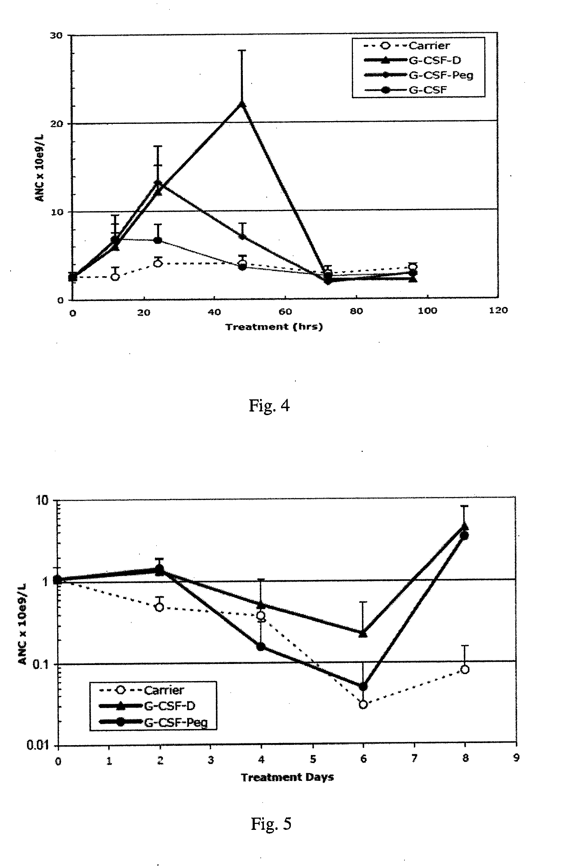

Fig. 4 shows the effect of single injection of rhG-CSF monomer (G-CSF and pegylated G-CSF (G-CSF-Peg)) and G-CSF dimer at equal molar dosage on the neutrophil count in the peripheral blood of healthy mice (average value ± standard deviation). The result indicated that the G-CSF dimer of the present invention had a stronger *in vivo* effect of driving the differentiation and maturation of myeloid hematopoietic cells, increasing the absolute neutrophil count (ANC) in the peripheral blood.

Fig. 5 shows the effect of G-CSF monomer and G-CSF dimer at equal molar dosage on the neutrophil count in mice model with 5-FU-induced neutropenia. The result indicated that the G-CSF dimer (G-CSF-D) of the present invention had a better therapeutic effect than pegylated G-CSF monomer.

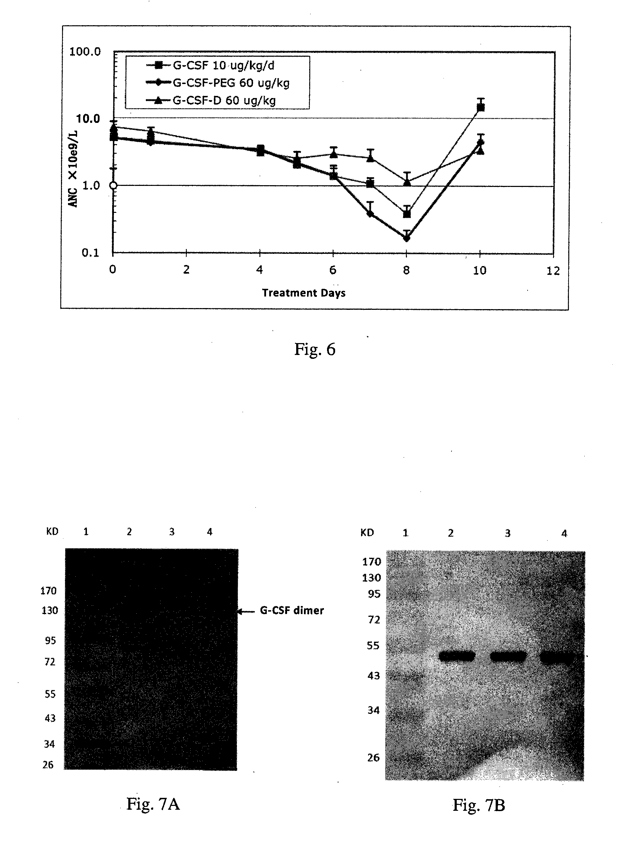

Fig. 6 shows the effect of G-CSF monomer and G-CSF dimer in cynomolgus monkeys model with cyclophosphamide-induced neutropenia. The result indicated that the G-CSF dimer (G-CSF-D) of the present invention had a better therapeutic effect than pegylated G-CSF monomer.

Fig. 7A shows that under non-reducing conditions, the immunoblot analysis results (Western blot) of cell culture supernatant, purified intermediate product and purified G-CSF dimer, using anti-human G-CSF monoclonal antibody (R&D systems, Cat.MAB214) as the first antibody and horseradish peroxidase-labeled anti-mouse IgG goat antibody as the second antibody. The lanes are as follow: 1. molecular weight standards; 2. cell culture supernatants; 3. purified intermediate product; and 4. purified G-CSF dimer.

Fig. 7B shows that under reducing conditions, the immunoblot analysis results (Western blot) of cell culture supernatant, purified intermediate product and purified G-CSF dimer, using anti-human G-CSF monoclonal antibody (R&D systems, Cat.MAB214) as the first antibody and horseradish peroxidase-labeled anti-mouse IgG goat antibody as the second antibody. The lanes are as follow: 1. molecular weight standards; 2. cell culture supernatants; 3. purified intermediate product; and 4. purified G-CSF dimer (The molecular weight of G-CSF-Fc monomer is around 48 KD).

### DETAILED DESCRIPTION OF THE PREFERRED EMBODIMENTS

Upon an extensive and thorough research, the inventors have, for the first time ever, accidentally discovered that on comparing with G-CSF monomer, rhG-CSF dimer can generate a stronger receptor activation signal to accelerate the differentiation and proliferation of bone marrow neutrophils. Meanwhile, the properties of the pharmacokinetics and pharmacodynamics of G-CSF dimer are better than that of the rhG-CSF monomer. Therefore, the G-CSF dimer can effectively reduce the extent of the severe neutropenia and shorten the time of severe neutropenia for cancer patients upon receiving chemotherapy. The present invention was made based on the above understanding.

**G-CSF dimer**

The first embodiment of the present invention is a G-CSF dimer represented by the aforesaid formula (I) and the structural illustration thereof is shown in Figs. 1-3. In these figures, carrier protein includes but not limited to Fc fragment of human IgG1, IgG2, IgG3 and IgG4, and human albumin.

In one preferred embodiment, G-CSF can be disposed at the C-terminal or N-terminal of the carrier protein.

As used herein, "linker" can refer to a short peptide for connecting the two G-CSF monomers and being disposed therebetween. There is no special restriction on the length of the linker. A linker is usually 5-50 amino acid residues in length; in general, a linker does not affect or significantly affect the proper fold and spatial conformation formed by the configuration of the two G-CSF monomers. Examples of linker include but not limited to:

In a further preferred embodiment, the linker comprises amino acid sequence selected from a group consisting of:

(a). an amino acid sequence with 3-16 amino acid residues formed by hydrophobic amino acids glycine (Gly) or proline (Pro), such as Gly-Pro-Gly-Pro-Gly-Pro;

(b). an amino acid sequence encoded by multiple cloning sites. Such sequence usually contains 5-20 amino acid residues; in a preferred embodiment, such sequence contains 10-20 amino acid residues;

(c). an amino acid sequence comprising protein(s) not from G-CSF monomer, such as an amino acid sequence of IgG or albumin;

(d). an amino acid sequence comprising any combination of (a), (b), and (c) above.

In one preferred embodiment, the linker has the sequence of GSGGGSGGGGSGGGGS (i.e. 175-190 amino acid residues of SEQ ID NO:1). In another preferred embodiment, the linker has the sequence of ASTKGP (i.e. 175-180 amino acid residues of SEQ ID NO:3).

In a further preferred embodiment, an amino acid sequence not affecting the biological activity of G-CSF monomer can be added to the N-terminal or C-terminal of the fusion protein. In a preferred embodiment, such appended amino acid sequence is beneficial to expression *(e.g.* signal peptide), purification *(e.g.* 6xHis sequence, the cleavage site of *Saccharomyces cerevisiae α-*factor signal peptide (Glu-Lys-Arg)), or enhancement of biological activity of the fusion protein.

**Preparation Method**

The encoding of the DNA sequence of the G-CSF dimer or fusion protein of the present invention can be entirely synthesized artificially. Alternatively, the encoded DNA sequences of the first G-CSF monomer and/or the second G-CSF monomer can be obtained by PCR amplification or synthesis and then joined together to form the encoded DNA sequence of the G-CSF dimer or fusion protein of the present invention.

In order to enhance the expression volume of the host cells, modification can be performed on the encoded sequence of G-CSF dimer. For example, codon bias of host cells can be used to eliminate sequences that are not beneficial to gene transcription and translation. In a preferred embodiment, codon bias of yeast cells or mammalian cells can be used together with DNA software for detecting genes of G-CSF dimer, in order to eliminate sequences that are not beneficial to gene transcription and translation. In one preferred embodiment, the eliminated sequences can be intron cutting site, transcription terminating sequence, etc.

After the encoded DNA sequence of the novel fusion protein of the present invention is obtained, it is first inserted into an appropriate expression carrier, followed by an appropriate host cell. Finally, the transformed host cell is cultivated and purified to obtain the novel fusion protein of the present invention.

As used herein and in the claims, "carrier" refers to plasmid, cosmid, expression vehicle, cloning vector, virus vector, etc.

In this invention, carrier known in the art, such as those available in the market, can be used. For example, with the use of carrier obtained from the market, encoded nucleotide sequence of the novel fusion protein of the present invention is operationally connected to the expressing and controlling sequence to form the protein-expressing carrier.

As used herein and in the claims, "operationally connected" refers to a scenario that some parts of a linear DNA sequence can affect the biological activity of other parts of the same linear DNA sequence. For instance, if signal DNA is used as the expression of a precursor and participates in secretion of polypeptides, the signal DNA (secretion leader sequence) is "operationally connected" to the polypeptides. If a promoter controls sequence transcription, the promoter is "operationally connected" to the encoded sequence. If a ribosome binding site is situated at a position where translation thereof is made possible, the ribosome binding site is "operationally connected" to the encoded sequence. In general, "operationally connected" means that the residues of concern are in proximity; for secretion leader sequence, "operationally connected" refers to proximity within the reading frame.

As used herein and in the claims, "host cells" refers to both prokaryotic cells and eukaryotic cells. Prokaryotic host cells commonly used include *E*. *coli, B. subtilis,* etc. Eukaryotic host cells commonly used include yeast cells, insect cells, mammalian cells, etc. In a preferred embodiment, the host cells used are eukaryotic cells; in another preferred embodiment, the host cells used are mammalian cells.

After the transformed host cells are obtained, they can be cultivated under an environment suitable to express the fusion protein of the present invention for expressing the fusion protein. The expressed fusion protein is then separated.

**Pharmaceutical Composition and Method Of Administration Thereof**

Since the G-CSF dimer of the present invention can generate a stronger receptor activation signal and has an excellent serum half-life, the G-CSF dimer and a pharmaceutical composition comprising the G-CSF dimer as the main active ingredient can be used for treating neutropenia. In a preferred embodiment, the neutropenia comprises a condition in which neutropenia is induced by chemotherapy and/or radiotherapy.

The pharmaceutical composition of the present invention comprises a safe and effective amount of the G-CSF dimer of the present invention and a pharmaceutically acceptable excipient or carrier. "Safe and effective amount" refers to an amount of a compound sufficient to substantially improve the condition of the patient in need thereof without causing serious side-effects. In general, the pharmaceutical composition comprises 0.001-1,000 mg of G-CSF dimer of the present invention per dose; in a preferred embodiment, the pharmaceutical composition comprises 0.05-300 mg of G-CSF dimer of the present invention per dose; in a further preferred embodiment, the pharmaceutical composition comprises 0.5-200 mg of G-CSF dimer of the present invention per dose.

The compound of the present invention and its pharmaceutically acceptable salts can be manufactured into different formulations, which comprises a safe and effective amount of the G-CSF dimer of the present invention or its pharmaceutically acceptable salts and a pharmaceutically acceptable excipient or carrier. "Safe and effective amount" refers to an amount of a compound sufficient to substantially improve the condition of the patient in need thereof without causing serious side-effects. The safe and effective amount of a compound is determined according to the age, condition, course of treatment, etc. of the patient in treatment.

"Pharmaceutically acceptable excipient or carrier" refers to solid or liquid filling or gelatin materials with one or different kinds of compatibility which are suitable to be used in human with sufficient purity and sufficiently low toxicity. "Compatibility" refers to the ability of each ingredient of the composition to mutually blend with the compound of the present invention and the mutual blending ability there between, without substantially decreasing the clinical efficacy of the compound. Some of the examples of pharmaceutically acceptable excipient or carrier include cellulose and its derivatives (e.g. sodium carboxymethylcellulose, sodium ethylcellulose, cellulose acetate, etc), gelatin, speckstone, solid lubricating agent (e.g. stearic acid, magnesium stearate), calcium sulphate, plant oil (e.g. pea oil, sesame oil, peanut oil, olive oil, etc.), polyols (e.g. propylene glycol, glycerol, mannitol, sorbitol, etc.), emulsifier (e.g. Tween®), wetting agent (e.g sodium lauryl sulfate), colorant, flavoring agent, stabilizer, anti-oxidant, antiseptic, pyrogen-free water, etc.

Route of administration of the G-CSF dimer of the present invention comprises oral administration, rectal administration, parenteral administration (intravenous, intramuscular, or subcutaneous), and partial administration.

Solid form for oral administration comprises capsules, tablets, pills, powder, and granules. In these solid forms, active compound is mixed with at least one of the conventionally inert excipients (or carriers), such as sodium citrate, dicalcium phosphate, or any of the following ingredients: (a) filing or bulking agent, e.g. starch, lactose, sucrose, glucose, mannitol, and silicic acid; (b) adhesion agent, e.g. carboxymethylcellulose, alginate, gelatin, polyvinyl pyrrolidone, sucrose, and acacia; (c) humectants, e.g. glycerol; (d) disintegrating agent, e.g. agar, calcium carbonate, potato starch or cassava starch, alginic acid, compounded silicate, and sodium carbonate; (e) buffering agent, e.g. paraffin wax; (f) absorption accelerating agent, e.g. quaternary amine compound; (g) wetting agent, e.g. cetanol and glycerin monostearate; (h) absorbent, e.g. bolus alba; and (i). lubricating agent, e.g. speckstone, calcium stearate, sodium stearate, solid polyethylene glycol, sodium lauryl sulfate, or any mixture thereof. Capsules, tablets, and pills can also comprise buffering agent.

Solid forms such as tablets, sugar pill, capsules, pills, and granules can be prepared with coating and core-shell materials, such as casing and other materials known in the art. These materials comprise opacifying agent and the active compound or compound in such composition can be released in a delayed fashion that the release is done in certain part of the alimentary canal. Embedding component such as polymer materials and wax materials can be used. If desired, active compounds can be mixed with one or more of the above-described excipients to formulate a micro capsule form.

Liquid forms for oral administration comprise pharmaceutically acceptable emulsion, solution, suspension, syrup, or tincture. Apart from active compounds, liquid forms also comprise inert diluents conventionally used in the art such as water or other solvent, solublilizing agent and emulsifier such as ethanol, isopropanol, carbonate acetate, ethyl acetate, propan-2-ol, 1,3-butan-2-ol, dimethylfomamide, and oil, in particular cotton oil, peanut oil, castor oil, olive oil, maize embryo oil, and sesame oil or any mixture thereof.

Apart from the inert diluents, the compound can also comprise additives, such as wetting agent, emulsifying agent, suspending agent, sweetening agent, correctives, and spices.

Apart from active compounds, suspension can also comprise suspending agent, such as ethoxyl isostearic alcohol, polyoxyethylene sorbitol, sorbitan, microcrystalline cellulose, aluminium methoxide, agar, or any mixture thereof.

Compounds used for parenteral administration can also comprise physiologically acceptable sterile water or anhydrous solution, dispersion solution, suspension, or emulsion, and sterile powder that can be re-dissolved into sterile injectable solution or dispersion solution. Appropriate hydrated or anhydrous carriers, diluting agent, solvent, or excipient comprises water, ethanol, polyols, and their appropriate mixtures thereof.

Forms of the G-CSF dimer of the present invention used for partial administration comprise ointment, powder, patch, sprayer, and inhalant. Under sterile conditions, active components can be mixed with physiologically acceptable carrier and any antiseptic, buffering agent, or may be propellant if desired.

The G-CSF dimer of the present invention can be solely administrated or be administrated in conjunction with any pharmaceutically acceptable compounds. Usually, the G-CSF dimer of the present invention is not administrated associated with a G-CSF monomer.

On using the pharmaceutical composition, a safe and effective of the amount of the G-CSF dimer of the present invention is administrated to a mammal (e.g. human) in use thereof in which the dosage administrated is a pharmaceutically acceptable effective administration dosage. For a human of 60kg, the administration dosage is usually 0.01-300 mg; in a preferred embodiment, the administration dosage is 0.5-100 mg. In determination of the actual dosage, factors known in the art such as administration route, patients' condition, etc. have to be considered, which is clear to a skilled person in the art.

There are many advantages of the G-CSF dimer of the present invention which include but not limited to:

1. A stronger receptor activation signal;

2. A longer *in vivo* biological half-life.

3. A substantial clinical efficacy in the treatment of neutropenia in animal models, which is better than other present products.

The following exemplary embodiments further describe the present invention. Although the description referred to particular embodiments, it will be clear to one skilled in the art that the present invention may be practiced with variation of these specific details. Hence this invention should not be construed as limited to the embodiments set forth herein. Further, for the embodiments in which details of the experimental methods are not described, such methods are carried out according to conventional conditions such as those described in Sambrook et al. Molecular Cloning: A Laboratory Manual (New York: Cold Spring Harbor Laboratory Pres, 1989), or suggested by the manufacturers.

**Example 1**

The G-CSF dimer with the structure described in Figs. 1-3 is prepared and purified by conventional methods. SEQ ID NO:1 represents G-CSF dimer and SEQ ID NOs:2-5 represent G-CSF monomer.

**Example** 2 - ***In vivo* half-life of G-CSF dimer**

Rats received a single subcutaneous injection of G-CSF dimer (which is formed by two G-CSF monomers of SEQ ID NO: 2) with a dose of 100 µg/kg. The pharmacokinetic parameters (n=6) were calculated and listed in Table 1 below. The half-life of G-CSF monomer in rats is about 2 hours.

| Parameter | Unit | Average Value | SD |
|---|---|---|---|
| AUC₍₀₋ₜ₎ | ng/mL * h | 4234.8 | 640.3 |
| MRT₍₀₋ₜ₎ | h | 21.6 | 1.4 |
| t_{(1/2)} | h | 7.7 | 1.2 |
| Clz/F | L/h/kg | 0.024 | 0.003 |
| Cmax | ng/mL | 162.2 | 30.2 |

**Example 3 -- The effect of G-CSF monomer and G-CSF dimer at equal molar dosage on proliferation of the neutrophil in healthy mice (G-CSF dimer can generate a stronger receptor activation signal *in vivo*)**

ICR mice, female, 20-22 grams, were randomly divided into four groups with 6-8 mice per group. The injection volume is 0.1 ml/10g of body weight, and each test group were given equal molar dosage of the G-CSF molecule (i.e. 1 mole of G-CSF dimer comprises 2 moles of G-CSF monomer). In other words, the mice were injected subcutaneously once with an equal volume of the carrier (control group), G-CSF-Peg 40 *µ*g/kg ^{,} rhG-CSF 40 µg/kg and G-CSF-D 100 µg/kg.

G-CSF-Peg monomer is Neulasta (Amgen, Pegylated Filgrastim).

G-CSF monomer is injectable rhG-CSF (GenSci).

G-CSF-D is a G-CSF dimer formed by two G-CSF monomers with an amino acid sequence as shown in SEQ ID NO:5.

After drug administration, blood samples (40 µL) were collected from orbital venous plexus at corresponding time points, and the blood count was detected and classified.

The result is shown in Fig. 4. On comparing with the control group, G-CSF monomer (rhG-CSF and G-CSF-Peg) and G-CSF dimer (G-CSF-D) have different effects in driving neutrophil hyperplasia in healthy mice. As mentioned, the tested animals were healthy animals, therefore after drug administration, the G-CSF monomer or G-CSF dimer bound with neutrophil receptor and then was metabolized and eliminated, which cause a decrease of the neutrophil count correspondingly. In the present test, the neutrophil counts of all three groups with G-CSF monomer or G-CSF dimer were returned to the baseline level in 72 hours.

It is worth noting that, at an equal molar dosage of the G-CSF molecule, the ANC increasing of G-CSF-D group was the highest. In particular, at the 48^{th} hour after drug administration, the average value of neutrophil of the G-CSF-D group, the G-CSF-Peg group and the G-CSF monomer group was 22.14 X10⁹/L,7.04 X10⁹/L and 3.61 X10⁹/L respectively, which means, the neutrophil count of the G-CSF-D group was 3.1 times of that of the G-CSF-Peg group, and 6.1 times of that of the G-CSF monomer group. Efficacy comparison result was: G-CSF-D > G-CSF-Peg > G-CSF, showing that G-CSF dimer has a better therapeutic effect. In other words, the increase of neutrophil in mice injected with G-CSF dimer was not only significantly higher than the group injected with G-CSF monomer, but also significantly higher than the group injected with G-CSF-Peg monomer which had a longer half-life. The result showed that G-CSF dimer has better biologic activities than G-CSF monomer in animals at equal molar dosage of G-CSF.

**Example 4 -- The effect of G-CSF monomer and G-CSF dimer at equal molar dosage on proliferation of the neutrophil in mice model with 5-FU-induced neutropenia (the therapeutic effect of G-CSF dimer in mice model)**

ICR mice, half male and half female, 24-26 grams, were randomly divided into groups with 10 mice per group. All animals were intravenously injected a dose of 150 mg/kg 5-FU for modeling. 24 hours later, animals of the control group were injected with the carrier; those of the G-CSF-D (dimer) group were injected with 1500 µg/kg rhG-CSF dimer (which was formed by two G-CSF monomers with amino acid sequence shown in SEQ ID NO:3); and those of the G-CSF-Peg monomer group were injected with 300 µg/kg Neulasta. The animals of the test groups were given at equal molar dosage of the G-CSF molecule (i.e. 1 mole of G-CSF dimer comprises 2 moles of G-CSF monomer) by subcutaneous injection. Each group was administrated once on Day 1, 3, 5 and 7 with an injection dosage of 0.1 ml/10g body weight. Peripheral blood samples were collected on Day 0, 2, 4, 6 and 8, and the white blood cell count was detected and classified.

The result was shown in Fig. 5. After the administration of 5-Fu, neutrophil count of the control group declined rapidly and the nadir was shown on Day 6. For animals with injection of rhG-CSF monomer (G-CSF-Peg) and G-CSF dimer (G-CSF-D) at equal molar dosage, the nadir of neutrophil counts was also shown on around Day 6. However, the neutrophil count of the G-CSF-D group (with an average value of 0.23X10⁹/L) was 4.6 times of that of the G-CSF-Peg group (with an average value of 0.05 X10⁹/L).

The result indicated that in the treatment of mice model with 5-Fu induced neutropenia, G-CSF dimer (G-CSF-D) had a significantly better therapeutic effect comparing to G-CSF monomer (G-CSF-Peg).

**Example 5 The effect of G-CSF monomer and G-CSF dimer on cyclophosphamide-induced neutropenia in cynomolgus monkeys model (the therapeutic effect of G-CSF dimer in cynomolgus monkeys model)**

24 healthy cynomolgus monkeys, half male and half female, were intravenously injected a dose of 60 mg/kg cyclophosphamide (lot: 2008040921, Jiangsu Hengrui Medicine Co., Ltd.) twice (Day 0 and Day 1) to induce a decrease of white blood cells and neutrophils. The cynomolgus monkeys were randomly divided into three groups, half male and half female. Starting from Day 5, the test groups were separately subcutaneously injected with G-CSF dimer (60 µg/kg, which is equal to 0.67 µM/kg) (G-CSF-D is a dimer formed by two G-CSF monomers with an amino acid sequence shown in SEQ ID NO:2) once; or with G-CSF-Peg monomer (60 µg/kg, which is equal to 3.2 µM/kg) once; or with G-CSF monomer (10 µg/kg/day, which is equal to 0.53 µ M/kg) for 5 continuous days with a total dose of 50 µg/kg, which is equal to 2.65 µM/kg. The injection volume was 0.2 mL/kg. Blood samples were collected at different time points, and the effect of F627 with different dose on the neutrophil count in peripheral blood of cynomolgus monkeys was observed.

The result was shown in Fig. 6. After the intravenous administration of cyclophosphamide, the neutrophil count of control group declined and reached the nadir on Days 6-8. For animals with injection equal dosage of rhG-CSF monomer and G-CSF-Peg monomer, the nadir of neutrophil count was reached on Days 6-7. For animals with single injection of G-CSF dimer, the occurrence of neutropenia was prevented when the molar dosage of the injected dimer was at 4.8 times less than that of G-CSF-Peg monomer. At the nadir (Day 8 after the administration of cyclophosphamide), the neutrophil count of the animal group injected with G-CSF dimer (average value 1.17 X 10⁹/L) once was 3.0 times of that with G-CSF monomer (average value 0.39×10⁹/L) and 6.9 times of that with G-CSF-Peg monomer (0.17×10⁹/L).

The result indicated that in the treatment of cynomolgus monkeys model with cyclophosphamide-induced neutropenia, G-CSF dimer (G-CSF-D) had a better therapeutic effect than G-CSF monomer and G-CSF-Peg, which can effectively prevent the occurrence of neutropenia induced by chemotherapy agent, and can reduce the severity of neutropenia and / or shorten the time of severe neutropenia.

**Example 6 - G-CSF dimer formed by G-CSF-Fc complex**

a. Construction of a cell line expressing G-CSF dimer

The full length cDNA sequence of the G-CSF-Fc complexes (as shown in SEQ ID NO: 7) was synthesized. cDNA sequence of human G-CSF monomer was connected with cDNA sequence of Fc fragment of IgG2. cDNA sequences containing Hind III site, and expression elements required by mammalian cell such as Kozak sequence and signal peptide were introduced at the 5' end. cDNA sequence containing EcoRI site was introduced at the 3' end. The full length G-CSF dimer cDNA sequence was cloned into pUC19 plasmid to obtain pG-CSF-Fc, transformed E. coli TG1. The plasmid was digested with Hind III and EcoRI, an approximately 1400bp G-CSF-IgG2Fc fragment was harvested and connected with pcDNA3 (Invitrogen) expression plasmid which was also digested with Hind III and EcoRI, and an expression plasmid pEX-G-CSF-Fc was then constructed. pEX-G-CSF-Fc was linearized, purified and transfected into CHO cells by electroporation. The transfected cells were selected in selecting media. The expression levels of individual clones were measured by ELISA. The cell lines with the higher protein expression levels were selected and cells thereof were frozen to generate cell bank.

The first G-CSF monomer synthesized using SEQ ID NO: 7 had a structure of "G-CSF-linker-IgG2Fc", and the amino acid sequence thereof was shown in SEQ ID NO: 6.

The expression plasmids were constructed in similar methods, resulting in the second, third and fourth G-CSF monomers (Table 1) with different structures. G-CSF dimers with different structures were obtained by similar expression methods.

**TABLE 1 Different G-CSF- Fc monomer**

| G-CSF-Fc monomer | Structure | Sequence of G-CSF | Sequence of linker | Sequence of IgG2Fc |
|---|---|---|---|---|
| No.1 | G-CSF-linker-IgG2Fc | SEQ ID NO.:1 amino acid residues 1-174 | SEQ ID NO.:1 amino acid residues 175-190 | SEQ ID NO.: 6 amino acid residues 191-413 |
| No.2 | | | SEQ ID NO.: 3 amino acid residues 175-180 | |
| No.3 | IgG2Fc-linker-G-CSF | | SEQ ID NO.:1 amino acid residues 175-190 | |
| No.4 | | | SEQ ID NO.: 3 amino acid residues 175-180 | |

b. Production of rhG-CSF dimer protein cells

One vial of cells (~ 1x 10⁷ cells/mL) from the cell bank was thawed and seeded in 10 mL basal medium in a 10 cm Petri dish, and incubated at 37°C, 5% CO₂ for approximately 24 hours.

The seeding expansion: The culture volume was expanded from 10 mL to 30-40 mL. When the cell density reached 1.0-1.5 x 10⁶ cells/mL with viability ≥ 90%, the culture volume was expanded to 300-400 mL progressively and then the culture was moved to shaking flasks and incubated at 120 rpm, 37°C, 5% CO₂.

Culture expansion in bioreactor (3L-10L): When the cell density in the seeding expansion reached 1.0 - 3.0 x 10⁶ cells/mL with viability ≥ 90%, the 300-400 mL seeding expanded culture was transferred to a 3-10 L bioreactor containing basal medium, with the culture control conditions at pH of 6.8, dissolved oxygen at approximately 50 % and stirring speed at 65-100 rpm.

Culture expansion in bioreactor (30 -100 L): When the cell density in the 3.0-10 L bioreactor reached 1.0 - 3.0 x 10⁶ cells/ml with viability ≥ 90%, the culture was transferred to 30 - 100 L bioreactor with the culture control conditions at pH of 6.8, dissolved oxygen at approximately 50 % and stirring speed at 65-100 rpm. The culture was fed for 12 to 48 hours before controlling the glucose level (<1g/L) with the addition of fed-batch medium.

c. Separation and purification of recombinant human G-CSF dimer protein

After the culture expansion in bioreactor, cell supernatant was harvested which contained G-CSF-Fc complex, G-CSF dimer, G-CSF-Fc multi-mers, and metabolites. After being harvested from the bioreactor culture, the cell culture supernatant was obtained by filtration and purified by a series of chromatography methods; for example, using a rProtein A Sepharose FF (GE Healthcare, cat#17-1279-04), eluted with 50 mM critic acid/ sodium citrate and 0.2M NaCl at pH 3.7-3.8, resulting in >90% pure G-CSF dimer. Additional chromatography steps were performed using Capto Adhere column with elution buffer of 50 mM NaAc/HAC and 0.2 M NaCl at pH 4.5-5.0, followed by SP Sepharose FF (GE Heathcare Cat #17-0729-04) and balanced with equilibrium buffer of 10mM PB (pH 6.0±0.1). Elution buffer used was 10mM PB and 0.2M NaCl (pH 7.2±0.1). Additional processes involved viral activation at low pH, filtration, resulting in G-CSF dimer.

The purity of the G-CSF dimer was > 95% (analyzed by reverse phase HPLC), with estimated molecular weight of 47±5 kD (analyzed by reduced SDS-PAGE analysis). The G-CSF dimer was glycosylated with oligosaccharide of 2-10% of the total molecular weight. The isoelectric point of the protein was between pH 5.8 to pH 6.8. The maximum UV absorbing wavelength was at 280 nM. The G-CSF dimer can activate STAT3 in M-NSF-60 cells and stimulate the proliferation of M-NSF-60 cells *in vitro* (the ED50 thereof was between 0.1-10 ng/mL).

The purification result of G-CSF dimer (comprising two G-CSF monomers with sequence shown in SEQ ID NO: 6) was shown in Fig. 7A and Fig. 7B, indicating G-CSF dimer was obtained after the purification (as shown in Fig 7A, lane 4).

Moreover, the results showed that, if the Fc fragment of the carrier protein contained four cysteine at the N-terminal, for example, ERKCCVECPPC(SEQ ID NO.: 2, amino acid residues 191-201), the formation of normally paired G-CSF dimer may be effected, indicating that it would be better for two Fc fragments to be connected with each other via 2-3 disulfide bonds.

**Example 7 - Pharmacokinetic properties of G-CSF dimer in human**

Healthy subjects were randomly divided into four dosage groups of 30, 60, 120, 240 µg/kg respectively receiving a single dose of 30, 60, 120, 240 µg/kg of G-CSF dimer (comprising two G-CSF monomers with sequence shown in SEQ ID NO: 6). Blood samples were collected at the 0.5, 1^{st}, 2^{nd} 4^{th}, 8^{th}, 16^{th} 24^{th}, 36^{th}, 48^{th}, 72^{nd}, 96^{th} hour, Day 6 (120 hours), 7, 9, 11, 13, and 15 after administration. Serum was separated and stored below -70°C. The blood concentrations of G-CSF-D were measured by ELISA (ELISA, Quantikine human G-CSF ELISA kit, R&D System, Inc. Minneapolis, Min, Cat: PDCS50). The pharmacokinetic parameters were calculated using the standard non-compartmental analytical procedures (Software WinNonlin v 5.2, Pharsight Corporation, USA). The results were shown in Table 2.

**TABLE 2**

| Parameter (n=6) | 30µg/kg | 60µg/kg | 120µg/kg | 240µg/kg |
|---|---|---|---|---|
| Cₘₐₓ (ng/mL) | 21.3 (10.3) | 44.6 (17.7) | 219.9 (76.6) | 759 (160) |
| Tₘₐₓ (h, median & range)* | 8 (8-16) | 8 (8-16) | 16 (16-36) | 36 (36) |
| t_{1/2} (h) | 43.9 (4.3) | 56.1 (23.3) | 59.3 (23.5) | 62.8 (10.8) |
| AUC_{(0-inf)} (ng.h/mL) | 778 (213) | 1847 (686) | 8349 (2769) | 46664 (17258) |
| CL/F (mL/h/kg) | 41.4 (12.8) | 36.8 (14.6) | 18.5 (7.7) | 5.7 (2.0) |

| | | | | |
|---|---|---|---|---|
| *Tₘₐₓ is represented by the median and rage values: data in the brackets is range values, and that outside the brackets is median values. | | | | |

All references mentioned in the present invention are cited herein by reference. Although the description referred to particular embodiments, it will be clear to one skilled in the art that the present invention may be practiced with variation of these specific details. Hence this invention should not be construed as limited to the embodiments set forth herein.

## Claims

1. Use of human granulocyte colony-stimulating factor (G-CSF) dimer in the manufacture of a drug for treating neutropenia.

2. The use of claim 1, wherein said neutropenia comprises a condition in which said neutropenia is induced by chemotherapy and/or radiotherapy.

3. The use of claim 1, wherein said human G-CSF dimer is shown as formula (I):
(I) M1-L-M2
wherein
M1 is a first human G-CSF monomer;
M2 is a second human G-CSF monomer; and
L is a linker connecting said first monomer and said second monomer and disposed therebetween,
said G-CSF dimer retains the biological activity of a G-CSF monomer and has a serum half-life of at least twice of the half-life of either said first or said second monomer.

4. The use of claim 3, wherein said linker L is selected from the group consisting of:
i). a short peptide comprising 3 to 50 amino acids; and
ii). a polypeptide of formula (II):
(II) -Z-Y-Z-
wherein
Y is a carrier protein;
Z is null, or a short peptide(s) comprising 1 to 30 amino acids;
"-" is a chemical bond or a covalent bond.

5. The use of claim 3, wherein said first monomer and said second monomer are of the same entity.

6. The use of claim 3, wherein said biological activity comprises:
(a). acting on neutrophil precursor cells and myeloid stem cells to drive the differentiation, development, and maturation of neutrophils; and
(b). activating mature neutrophils to participate in immune response.

7. The use of claim 4, wherein said carrier protein is albumin or Fc fragment of human IgG.

8. The use of claim 4, wherein said "-" is a peptide bond.

9. The use of claim 4, wherein said G-CSF dimer is formed by monomers in which said monomer comprises an amino acid sequence selected from a group consisting of SEQ ID NOs: 2-6.

10. A human G-CSF dimer of formula (I):
(I) M1-L-M2
wherein
M1 is a first G-CSF monomer;
M2 is a second G-CSF monomer; and
L is a linker connecting said first monomer and said second monomer and disposed therebetween,
said G-CSF dimer retains the biological activity of a G-CSF monomer and has a serum half-life of at least twice of the half-life of either said first or said second monomer.


SEQUENCE LISTING

<110>  GENERON (SHANGHAI) CORPROATION LTD.

<120>  USE OF G-CSF DIMER FOR THE TREATMENT OF NEUTROPENIA

<130>  P2011-0359

<150>  CN201010268290.X
<151>  2010-08-31

<160>  7

<170>  PatentIn version 3.5

<210>  1
<211>  364
<212>  PRT
<213>  Artificial sequence


<220>
<221>  misc_feature
<223>  G-CSF Dimer

<400>  1

Thr Pro Leu Gly Pro Ala Ser Ser Leu Pro Gln Ser Phe Leu Leu Lys
1               5                   10                  15


Cys Leu Glu Gln Val Arg Lys Ile Gln Gly Asp Gly Ala Ala Leu Gln
            20                  25                  30


Glu Lys Leu Cys Ala Thr Tyr Lys Leu Cys His Pro Glu Glu Leu Val
        35                  40                  45


Leu Leu Gly His Ser Leu Gly Ile Pro Trp Ala Pro Leu Ser Ser Cys
    50                  55                  60


Pro Ser Gln Ala Leu Gln Leu Ala Gly Cys Leu Ser Gln Leu His Ser
65                  70                  75                  80


Gly Leu Phe Leu Tyr Gln Gly Leu Leu Gln Ala Leu Glu Gly Ile Ser
                85                  90                  95


Pro Glu Leu Gly Pro Thr Leu Asp Thr Leu Gln Leu Asp Val Ala Asp
            100                 105                 110


Phe Ala Thr Thr Ile Trp Gln Gln Met Glu Glu Leu Gly Met Ala Pro
        115                 120                 125


Ala Leu Gln Pro Thr Gln Gly Ala Met Pro Ala Phe Ala Ser Ala Phe
    130                 135                 140

Gln Arg Arg Ala Gly Gly Val Leu Val Ala Ser His Leu Gln Ser Phe
145                 150                 155                 160

Leu Glu Val Ser Tyr Arg Val Leu Arg His Leu Ala Gln Pro Gly Ser
                165                 170                 175

Gly Gly Gly Ser Gly Gly Gly Gly Ser Gly Gly Gly Gly Ser Thr Pro
            180                 185                 190

Leu Gly Pro Ala Ser Ser Leu Pro Gln Ser Phe Leu Leu Lys Cys Leu
        195                 200                 205

Glu Gln Val Arg Lys Ile Gln Gly Asp Gly Ala Ala Leu Gln Glu Lys
    210                 215                 220

Leu Cys Ala Thr Tyr Lys Leu Cys His Pro Glu Glu Leu Val Leu Leu
225                 230                 235                 240

Gly His Ser Leu Gly Ile Pro Trp Ala Pro Leu Ser Ser Cys Pro Ser
                245                 250                 255

Gln Ala Leu Gln Leu Ala Gly Cys Leu Ser Gln Leu His Ser Gly Leu
            260                 265                 270

Phe Leu Tyr Gln Gly Leu Leu Gln Ala Leu Glu Gly Ile Ser Pro Glu
        275                 280                 285

Leu Gly Pro Thr Leu Asp Thr Leu Gln Leu Asp Val Ala Asp Phe Ala
    290                 295                 300

Thr Thr Ile Trp Gln Gln Met Glu Glu Leu Gly Met Ala Pro Ala Leu
305                 310                 315                 320

Gln Pro Thr Gln Gly Ala Met Pro Ala Phe Ala Ser Ala Phe Gln Arg
                325                 330                 335

Arg Ala Gly Gly Val Leu Val Ala Ser His Leu Gln Ser Phe Leu Glu
            340                 345                 350

Val Ser Tyr Arg Val Leu Arg His Leu Ala Gln Pro
        355                 360

<210>  2
<211>  418
<212>  PRT
<213>  Artificial sequence

<220>

<221>  misc_feature
<223>  G-CSF monomer with Fc fragment

<400>  2

Thr Pro Leu Gly Pro Ala Ser Ser Leu Pro Gln Ser Phe Leu Leu Lys
1               5                   10                  15

Cys Leu Glu Gln Val Arg Lys Ile Gln Gly Asp Gly Ala Ala Leu Gln
            20                  25                  30

Glu Lys Leu Cys Ala Thr Tyr Lys Leu Cys His Pro Glu Glu Leu Val
        35                  40                  45

Leu Leu Gly His Ser Leu Gly Ile Pro Trp Ala Pro Leu Ser Ser Cys
    50                  55                  60

Pro Ser Gln Ala Leu Gln Leu Ala Gly Cys Leu Ser Gln Leu His Ser
65                  70                  75                  80

Gly Leu Phe Leu Tyr Gln Gly Leu Leu Gln Ala Leu Glu Gly Ile Ser
                85                  90                  95

Pro Glu Leu Gly Pro Thr Leu Asp Thr Leu Gln Leu Asp Val Ala Asp
            100                 105                 110

Phe Ala Thr Thr Ile Trp Gln Gln Met Glu Glu Leu Gly Met Ala Pro
        115                 120                 125

Ala Leu Gln Pro Thr Gln Gly Ala Met Pro Ala Phe Ala Ser Ala Phe
    130                 135                 140

Gln Arg Arg Ala Gly Gly Val Leu Val Ala Ser His Leu Gln Ser Phe
145                 150                 155                 160

Leu Glu Val Ser Tyr Arg Val Leu Arg His Leu Ala Gln Pro Gly Ser
                165                 170                 175

Gly Gly Gly Ser Gly Gly Gly Gly Ser Gly Gly Gly Gly Ser Glu Arg
            180                 185                 190

Lys Cys Cys Val Glu Cys Pro Pro Cys Pro Ala Pro Pro Val Ala Gly
        195                 200                 205

Pro Ser Val Phe Leu Phe Pro Pro Lys Pro Lys Asp Thr Leu Met Ile
    210                 215                 220

Ser Arg Thr Pro Glu Val Thr Cys Val Val Val Asp Val Ser His Glu
225                 230                 235                 240

Asp Pro Glu Val Gln Phe Asn Trp Tyr Val Asp Gly Val Glu Val His
                245                 250                 255

Asn Ala Lys Thr Lys Pro Arg Glu Glu Gln Phe Asn Ser Thr Phe Arg
            260                 265                 270

Val Val Ser Val Leu Thr Val Val His Gln Asp Trp Leu Asn Gly Lys
        275                 280                 285

Glu Tyr Lys Cys Lys Val Ser Asn Lys Gly Leu Pro Ala Ser Ile Glu
    290                 295                 300

Lys Thr Ile Ser Lys Thr Lys Gly Gln Pro Arg Glu Pro Gln Val Tyr
305                 310                 315                 320

Thr Leu Pro Pro Ser Arg Glu Glu Met Thr Lys Asn Gln Val Ser Leu
                325                 330                 335

Thr Cys Leu Val Lys Gly Phe Tyr Pro Ser Asp Ile Ala Val Glu Trp
            340                 345                 350

Glu Ser Asn Gly Gln Pro Glu Asn Asn Tyr Lys Thr Thr Pro Pro Met
        355                 360                 365

Leu Asp Ser Asp Gly Ser Phe Phe Leu Tyr Ser Lys Leu Thr Val Asp
    370                 375                 380

Lys Ser Arg Trp Gln Gln Gly Asn Val Phe Ser Cys Ser Val Met His
385                 390                 395                 400

Glu Ala Leu His Asn His Tyr Thr Gln Lys Ser Leu Ser Leu Ser Pro
                405                 410                 415

Gly Lys

<210>  3
<211>  408
<212>  PRT
<213>  Artificial sequence

<220>
<221>  misc_feature
<223>  G-CSF monomer with Fc fragment

<400>  3

Thr Pro Leu Gly Pro Ala Ser Ser Leu Pro Gln Ser Phe Leu Leu Lys

1               5                   10                  15

Cys Leu Glu Gln Val Arg Lys Ile Gln Gly Asp Gly Ala Ala Leu Gln
            20                  25                  30

Glu Lys Leu Cys Ala Thr Tyr Lys Leu Cys His Pro Glu Glu Leu Val
        35                  40                  45

Leu Leu Gly His Ser Leu Gly Ile Pro Trp Ala Pro Leu Ser Ser Cys
    50                  55                  60

Pro Ser Gln Ala Leu Gln Leu Ala Gly Cys Leu Ser Gln Leu His Ser
65                  70                  75                  80

Gly Leu Phe Leu Tyr Gln Gly Leu Leu Gln Ala Leu Glu Gly Ile Ser
                85                  90                  95

Pro Glu Leu Gly Pro Thr Leu Asp Thr Leu Gln Leu Asp Val Ala Asp
            100                 105                 110

Phe Ala Thr Thr Ile Trp Gln Gln Met Glu Glu Leu Gly Met Ala Pro
        115                 120                 125

Ala Leu Gln Pro Thr Gln Gly Ala Met Pro Ala Phe Ala Ser Ala Phe
    130                 135                 140

Gln Arg Arg Ala Gly Gly Val Leu Val Ala Ser His Leu Gln Ser Phe
145                 150                 155                 160

Leu Glu Val Ser Tyr Arg Val Leu Arg His Leu Ala Gln Pro Ala Ser
                165                 170                 175

Thr Lys Gly Pro Glu Arg Lys Cys Cys Val Glu Cys Pro Pro Cys Pro
            180                 185                 190

Ala Pro Pro Val Ala Gly Pro Ser Val Phe Leu Phe Pro Pro Lys Pro
        195                 200                 205

Lys Asp Thr Leu Met Ile Ser Arg Thr Pro Glu Val Thr Cys Val Val
    210                 215                 220

Val Asp Val Ser His Glu Asp Pro Glu Val Gln Phe Asn Trp Tyr Val
225                 230                 235                 240

Asp Gly Val Glu Val His Asn Ala Lys Thr Lys Pro Arg Glu Glu Gln
                245                 250                 255

Phe Asn Ser Thr Phe Arg Val Val Ser Val Leu Thr Val Val His Gln
            260                 265                 270

Asp Trp Leu Asn Gly Lys Glu Tyr Lys Cys Lys Val Ser Asn Lys Gly
        275                 280                 285

Leu Pro Ala Ser Ile Glu Lys Thr Ile Ser Lys Thr Lys Gly Gln Pro
    290                 295                 300

Arg Glu Pro Gln Val Tyr Thr Leu Pro Pro Ser Arg Glu Glu Met Thr
305                 310                 315                 320

Lys Asn Gln Val Ser Leu Thr Cys Leu Val Lys Gly Phe Tyr Pro Ser
                325                 330                 335

Asp Ile Ala Val Glu Trp Glu Ser Asn Gly Gln Pro Glu Asn Asn Tyr
            340                 345                 350

Lys Thr Thr Pro Pro Met Leu Asp Ser Asp Gly Ser Phe Phe Leu Tyr
        355                 360                 365

Ser Lys Leu Thr Val Asp Lys Ser Arg Trp Gln Gln Gly Asn Val Phe
    370                 375                 380

Ser Cys Ser Val Met His Glu Ala Leu His Asn His Tyr Thr Gln Lys
385                 390                 395                 400

Ser Leu Ser Leu Ser Pro Gly Lys
                405

<210>  4
<211>  418
<212>  PRT
<213>  Artificial sequence

<220>
<221>  misc_feature
<223>  G-CSF monomer with Fc fragment

<400>  4

Glu Arg Lys Cys Cys Val Glu Cys Pro Pro Cys Pro Ala Pro Pro Val
1               5                   10                  15

Ala Gly Pro Ser Val Phe Leu Phe Pro Pro Lys Pro Lys Asp Thr Leu
            20                  25                  30

Met Ile Ser Arg Thr Pro Glu Val Thr Cys Val Val Val Asp Val Ser
        35                  40                  45

His Glu Asp Pro Glu Val Gln Phe Asn Trp Tyr Val Asp Gly Val Glu
    50                  55                  60

Val His Asn Ala Lys Thr Lys Pro Arg Glu Glu Gln Phe Asn Ser Thr
65                  70                  75                  80

Phe Arg Val Val Ser Val Leu Thr Val Val His Gln Asp Trp Leu Asn
                85                  90                  95

Gly Lys Glu Tyr Lys Cys Lys Val Ser Asn Lys Gly Leu Pro Ala Ser
            100                 105                 110

Ile Glu Lys Thr Ile Ser Lys Thr Lys Gly Gln Pro Arg Glu Pro Gln
        115                 120                 125

Val Tyr Thr Leu Pro Pro Ser Arg Glu Glu Met Thr Lys Asn Gln Val
    130                 135                 140

Ser Leu Thr Cys Leu Val Lys Gly Phe Tyr Pro Ser Asp Ile Ala Val
145                 150                 155                 160

Glu Trp Glu Ser Asn Gly Gln Pro Glu Asn Asn Tyr Lys Thr Thr Pro
                165                 170                 175

Pro Met Leu Asp Ser Asp Gly Ser Phe Phe Leu Tyr Ser Lys Leu Thr
            180                 185                 190

Val Asp Lys Ser Arg Trp Gln Gln Gly Asn Val Phe Ser Cys Ser Val
        195                 200                 205

Met His Glu Ala Leu His Asn His Tyr Thr Gln Lys Ser Leu Ser Leu
    210                 215                 220

Ser Pro Gly Lys Gly Ser Gly Gly Gly Ser Gly Gly Gly Gly Ser Gly
225                 230                 235                 240

Gly Gly Gly Ser Thr Pro Leu Gly Pro Ala Ser Ser Leu Pro Gln Ser
                245                 250                 255

Phe Leu Leu Lys Cys Leu Glu Gln Val Arg Lys Ile Gln Gly Asp Gly
            260                 265                 270

Ala Ala Leu Gln Glu Lys Leu Cys Ala Thr Tyr Lys Leu Cys His Pro
        275                 280                 285

Glu Glu Leu Val Leu Leu Gly His Ser Leu Gly Ile Pro Trp Ala Pro
    290                 295                 300

Leu Ser Ser Cys Pro Ser Gln Ala Leu Gln Leu Ala Gly Cys Leu Ser
305                 310                 315                 320

Gln Leu His Ser Gly Leu Phe Leu Tyr Gln Gly Leu Leu Gln Ala Leu
                325                 330                 335

Glu Gly Ile Ser Pro Glu Leu Gly Pro Thr Leu Asp Thr Leu Gln Leu
            340                 345                 350

Asp Val Ala Asp Phe Ala Thr Thr Ile Trp Gln Gln Met Glu Glu Leu
        355                 360                 365

Gly Met Ala Pro Ala Leu Gln Pro Thr Gln Gly Ala Met Pro Ala Phe
    370                 375                 380

Ala Ser Ala Phe Gln Arg Arg Ala Gly Gly Val Leu Val Ala Ser His
385                 390                 395                 400

Leu Gln Ser Phe Leu Glu Val Ser Tyr Arg Val Leu Arg His Leu Ala
                405                 410                 415

Gln Pro

<210>  5
<211>  408
<212>  PRT
<213>  Artificial sequence

<220>
<221>  misc_feature
<223>  G-CSF with Fc fragment

<400>  5

Glu Arg Lys Cys Cys Val Glu Cys Pro Pro Cys Pro Ala Pro Pro Val
1               5                   10                  15

Ala Gly Pro Ser Val Phe Leu Phe Pro Pro Lys Pro Lys Asp Thr Leu
            20                  25                  30

Met Ile Ser Arg Thr Pro Glu Val Thr Cys Val Val Val Asp Val Ser
        35                  40                  45

His Glu Asp Pro Glu Val Gln Phe Asn Trp Tyr Val Asp Gly Val Glu
    50                  55                  60

Val His Asn Ala Lys Thr Lys Pro Arg Glu Glu Gln Phe Asn Ser Thr

65                  70                  75                  80

Phe Arg Val Val Ser Val Leu Thr Val Val His Gln Asp Trp Leu Asn
                85                  90                  95

Gly Lys Glu Tyr Lys Cys Lys Val Ser Asn Lys Gly Leu Pro Ala Ser
            100                 105                 110

Ile Glu Lys Thr Ile Ser Lys Thr Lys Gly Gln Pro Arg Glu Pro Gln
        115                 120                 125

Val Tyr Thr Leu Pro Pro Ser Arg Glu Glu Met Thr Lys Asn Gln Val
    130                 135                 140

Ser Leu Thr Cys Leu Val Lys Gly Phe Tyr Pro Ser Asp Ile Ala Val
145                 150                 155                 160

Glu Trp Glu Ser Asn Gly Gln Pro Glu Asn Asn Tyr Lys Thr Thr Pro
                165                 170                 175

Pro Met Leu Asp Ser Asp Gly Ser Phe Phe Leu Tyr Ser Lys Leu Thr
            180                 185                 190

Val Asp Lys Ser Arg Trp Gln Gln Gly Asn Val Phe Ser Cys Ser Val
        195                 200                 205

Met His Glu Ala Leu His Asn His Tyr Thr Gln Lys Ser Leu Ser Leu
    210                 215                 220

Ser Pro Gly Lys Ala Ser Thr Lys Gly Pro Thr Pro Leu Gly Pro Ala
225                 230                 235                 240

Ser Ser Leu Pro Gln Ser Phe Leu Leu Lys Cys Leu Glu Gln Val Arg
                245                 250                 255

Lys Ile Gln Gly Asp Gly Ala Ala Leu Gln Glu Lys Leu Cys Ala Thr
            260                 265                 270

Tyr Lys Leu Cys His Pro Glu Glu Leu Val Leu Leu Gly His Ser Leu
        275                 280                 285

Gly Ile Pro Trp Ala Pro Leu Ser Ser Cys Pro Ser Gln Ala Leu Gln
    290                 295                 300

Leu Ala Gly Cys Leu Ser Gln Leu His Ser Gly Leu Phe Leu Tyr Gln
305                 310                 315                 320

Gly Leu Leu Gln Ala Leu Glu Gly Ile Ser Pro Glu Leu Gly Pro Thr
                325                 330                 335

Leu Asp Thr Leu Gln Leu Asp Val Ala Asp Phe Ala Thr Thr Ile Trp
            340                 345                 350

Gln Gln Met Glu Glu Leu Gly Met Ala Pro Ala Leu Gln Pro Thr Gln
        355                 360                 365

Gly Ala Met Pro Ala Phe Ala Ser Ala Phe Gln Arg Arg Ala Gly Gly
    370                 375                 380

Val Leu Val Ala Ser His Leu Gln Ser Phe Leu Glu Val Ser Tyr Arg
385                 390                 395                 400

Val Leu Arg His Leu Ala Gln Pro
                405

<210>  6
<211>  413
<212>  PRT
<213>  Artificial sequence

<220>
<221>  misc_feature
<223>  G-CSF monomer with Fc fragment

<400>  6

Thr Pro Leu Gly Pro Ala Ser Ser Leu Pro Gln Ser Phe Leu Leu Lys
1               5                   10                  15

Cys Leu Glu Gln Val Arg Lys Ile Gln Gly Asp Gly Ala Ala Leu Gln
            20                  25                  30

Glu Lys Leu Cys Ala Thr Tyr Lys Leu Cys His Pro Glu Glu Leu Val
        35                  40                  45

Leu Leu Gly His Ser Leu Gly Ile Pro Trp Ala Pro Leu Ser Ser Cys
    50                  55                  60

Pro Ser Gln Ala Leu Gln Leu Ala Gly Cys Leu Ser Gln Leu His Ser
65                  70                  75                  80

Gly Leu Phe Leu Tyr Gln Gly Leu Leu Gln Ala Leu Glu Gly Ile Ser
                85                  90                  95

Pro Glu Leu Gly Pro Thr Leu Asp Thr Leu Gln Leu Asp Val Ala Asp
            100                 105                 110

Phe Ala Thr Thr Ile Trp Gln Gln Met Glu Glu Leu Gly Met Ala Pro
        115                 120                 125

Ala Leu Gln Pro Thr Gln Gly Ala Met Pro Ala Phe Ala Ser Ala Phe
    130                 135                 140

Gln Arg Arg Ala Gly Gly Val Leu Val Ala Ser His Leu Gln Ser Phe
145                 150                 155                 160

Leu Glu Val Ser Tyr Arg Val Leu Arg His Leu Ala Gln Pro Gly Ser
                165                 170                 175

Gly Gly Gly Ser Gly Gly Gly Gly Ser Gly Gly Gly Gly Ser Val Glu
            180                 185                 190

Cys Pro Pro Cys Pro Ala Pro Pro Val Ala Gly Pro Ser Val Phe Leu
        195                 200                 205

Phe Pro Pro Lys Pro Lys Asp Thr Leu Met Ile Ser Arg Thr Pro Glu
    210                 215                 220

Val Thr Cys Val Val Val Asp Val Ser His Glu Asp Pro Glu Val Gln
225                 230                 235                 240

Phe Asn Trp Tyr Val Asp Gly Val Glu Val His Asn Ala Lys Thr Lys
                245                 250                 255

Pro Arg Glu Glu Gln Phe Asn Ser Thr Phe Arg Val Val Ser Val Leu
            260                 265                 270

Thr Val Val His Gln Asp Trp Leu Asn Gly Lys Glu Tyr Lys Cys Lys
        275                 280                 285

Val Ser Asn Lys Gly Leu Pro Ala Ser Ile Glu Lys Thr Ile Ser Lys
    290                 295                 300

Thr Lys Gly Gln Pro Arg Glu Pro Gln Val Tyr Thr Leu Pro Pro Ser
305                 310                 315                 320

Arg Glu Glu Met Thr Lys Asn Gln Val Ser Leu Thr Cys Leu Val Lys
                325                 330                 335

Gly Phe Tyr Pro Ser Asp Ile Ala Val Glu Trp Glu Ser Asn Gly Gln
            340                 345                 350

Pro Glu Asn Asn Tyr Lys Thr Thr Pro Pro Met Leu Asp Ser Asp Gly
        355                 360                 365

Ser Phe Phe Leu Tyr Ser Lys Leu Thr Val Asp Lys Ser Arg Trp Gln
    370                 375                 380

Gln Gly Asn Val Phe Ser Cys Ser Val Met His Glu Ala Leu His Asn
385                 390                 395                 400

His Tyr Thr Gln Lys Ser Leu Ser Leu Ser Pro Gly Lys
                405                 410

<210>  7
<211>  1353
<212>  DNA
<213>  Artificial sequence

<220>
<221>  misc_feature
<223>  DNA sequence encoding G-CSF monomer with Fc fragment

<400>  7
aagcttccca gacccatggc tggacctgcc acccagagcc ccatgaagct gatggccctg      60

cagctgctgc tgtggcacag tgcactctgg acagtgcagg aagccacccc cctgggccct     120

gccagctccc tgccccagag cttcctgctc aagtgcttag agcaagtgag gaagatccag     180

ggcgatggcg cagcgctcca ggagaagctg tgtgccacct acaagctgtg ccaccccgag     240

gagctggtgc tgctcggaca ctctctgggc atcccctggg ctcccctgag cagctgcccc     300

agccaggccc tgcagctggc aggctgcttg agccaactcc atagcggcct tttcctctac     360

caggggctcc tgcaggccct ggaagggatc tcccccgagt tgggtcccac cttggacaca     420

ctgcagctgg acgtcgccga ctttgccacc accatctggc agcagatgga agaactggga     480

atggcccctg ccctgcagcc cacccagggt gccatgccgg ccttcgcctc tgctttccag     540

cgccgggcag gaggggtcct ggttgcctcc catctgcaga gcttcctgga ggtgtcgtac     600

cgcgttctac gccaccttgc ccagcccgga tccggtggcg gttccggtgg aggcggaagc     660

ggcggtggag gatcagtcga gtgcccaccg tgcccagcac cacctgtggc aggaccgtca     720

gtcttcctct tccccccaaa acccaaggac accctcatga tctcccggac ccctgaggtc     780

acgtgcgtgg tggtggacgt gagccacgaa gaccccgagg tccagttcaa ctggtacgtg     840

gacggcgtgg aggtgcataa tgccaagaca aagccacggg aggagcagtt caacagcacg     900

ttccgtgtgg tcagcgtcct caccgttgtg caccaggact ggctgaacgg caaggagtac     960

aagtgcaagg tctccaacaa aggcctccca gcctccatcg agaaaaccat ctccaaaacc    1020

aaagggcagc cccgagaacc acaggtgtac accctgcccc catcccggga ggagatgacc    1080

aagaaccagg tcagcctgac ctgcctggtc aaaggcttct accccagcga catcgccgtg    1140

gagtgggaga gcaatgggca gccggagaac aactacaaga ccacacctcc catgctggac 1200

tccgacggct ccttcttcct ctacagcaag ctcaccgtgg acaagagcag gtggcagcag 1260

gggaacgtct tctcatgctc cgtgatgcat gaggctctgc acaaccacta cacgcagaag 1320

agcctctccc tgtctccggg taaatgagaa ttc 1353